# FASCICULE DE BREVET EUROPEEN

(11) **EP 4 191 225 B1**
(45) Date de publication et mention de la délivrance du brevet: **01.01.2025**
(21) Numéro de dépôt: 22207967.5
(22) Date de dépôt: 17.11.2022
(51) Int. Cl.: G01N 1/28, G01N 1/44, C12M 1/00

(54) **SYSTÈME AUTOMATISÉ ET PROCÉDÉ DE PRÉPARATION D'UN ÉCHANTILLON BIOLOGIQUE**
AUTOMATISIERTES SYSTEM ZUR VORBEREITUNG EINER BIOLOGISCHEN PROBE
AUTOMATED SYSTEM FOR PREPARING A BIOLOGICAL SAMPLE

(30) Priorité: 03.12.2021 FR 2112885
(43) Date de publication de la demande: 07.06.2023
(73) Titulaire: Commissariat à l'Energie Atomique et aux Energies Alternatives, 75015 Paris (FR)
(72) Inventeur: BOURDAT, Anne-Gaëlle, 38054 Grenoble cedex 09 (FR); BAQUE, Mélissa, 38054 Grenoble cedex 09 (FR); BORDY, Thomas, 38054 Grenoble cedex 09 (FR)
(74) Mandataire: INNOV-GROUP

(56) Documents cités:
- WO-A1-2015/181743
- US-A1- 2013 164 754
- US-A1- 2013 217 113
- US-A1- 2014 087 359
- US-A1- 2021 346 885

## Description

### Domaine technique de l'invention

La présente invention se rapporte à un système automatisé de préparation d'un échantillon biologique.

### Etat de la technique

La préparation d'un échantillon biologique passe souvent par une étape de lyse des espèces biologiques présentes dans l'échantillon. La lyse de cellules biologiques est souvent nécessaire pour récupérer et étudier le matériel intracellulaire, par exemple l'ADN de la cellule.

Les méthodes classiquement utilisées pour la lyse d'espèces biologiques sont les suivantes :
- La lyse chimique qui consiste à ajouter une solution chimique de lyse sur les espèces biologiques pour faire éclater les cellules ;
- La lyse par choc thermique qui consiste à faire subir un cycle de température aux espèces biologiques ;
- La lyse par champ électrique qui consiste à soumettre les espèces biologiques à un champ électrique ;
- La lyse mécanique qui consiste à venir broyer les espèces biologiques pour libérer le matériel biologique ;

Différentes solutions de lyse mécanique ont déjà été proposées dans l'état de la technique, notamment dans les demandes de brevets WO2015/181743A1 et EP3222989A1**.** Ces solutions consistent principalement à venir broyer les espèces biologiques contre une paroi rugueuse, ou par cisaillement. Un autre système de préparation d'un échantillon par lyse est divulgué dans US 2013/217113 A1.

Ces solutions antérieures ne sont cependant pas adaptées pour effectuer des lyses d'échantillons biologiques avec une cadence élevée, et éventuellement avec plusieurs échantillons en parallèle.

Le but de l'invention est de proposer un système capable de préparer des échantillons biologiques avec une cadence élevée, et éventuellement capable de traiter plusieurs échantillons en parallèle.

### Exposé de l'invention

Ce but est atteint par un système automatisé de préparation d'un échantillon biologique contenant des espèces biologiques selon la revendication 1, comportant :
- Une plaque support dans laquelle sont réalisés un ou plusieurs puits traversants,
- Chaque puits traversant présentant deux accès opposés, un premier accès et un deuxième accès, le premier accès étant séparé du deuxième accès par une paroi poreuse, formant un filtre, contre laquelle peut être déposé ledit échantillon biologique à lyser,
- Et pour chaque puits :
   ∘ Un premier organe mobile, actionnable en translation dans l'axe du puits pour s'insérer à travers son premier accès,
   ∘ Un deuxième organe mobile, actionnable en translation dans l'axe du puits pour s'insérer à travers le deuxième accès,
- Des moyens d'entraînement en translation de chaque premier organe mobile et de chaque deuxième organe mobile,
- Des moyens de stimulation de type mécanique et/ou thermique de chaque premier organe mobile et/ou de chaque deuxième organe mobile et/ou de la plaque support,
- Une unité de contrôle configurée pour :
   ∘ Commander lesdits moyens d'entraînement,
   ∘ Commander lesdits moyens de stimulation.

Selon une particularité, les moyens d'entraînement sont configurés pour déplacer ladite plaque support entre au moins deux positions distinctes, une position de stockage dans laquelle elle est en attente d'utilisation et une position de travail dans laquelle elle est positionnée pour recevoir dans chaque puits, un échantillon biologique.

Selon une autre particularité, le système comporte, pour chaque puits de la plaque support, des moyens de dispense d'un échantillon biologique dans chaque puits de la plaque de support, les moyens d'entraînement étant configurés pour déplacer les moyens de dispense entre deux positions distinctes, une position de stockage et une position de travail dans laquelle ils sont contrôlables pour injecter un échantillon biologique dans chaque puits.

Selon une autre particularité, les moyens de stimulation de type mécanique comprennent des moyens d'actionnement de type vibratoire coopérant avec le premier organe mobile et/ou le deuxième organe mobile.

Selon une autre particularité, les moyens de stimulation de type mécanique comprennent des moyens d'actionnement en rotation autour de son axe du premier organe mobile et/ou du deuxième organe mobile.

Selon une autre particularité, les moyens de stimulation de type thermique comportent un élément chauffant intégré dans le premier organe mobile et/ou le deuxième organe mobile.

Selon une autre particularité, le premier organe mobile et/ou le deuxième organe mobile présente une extrémité libre et le système comporte, pour le premier organe mobile et/ou le deuxième organe mobile, un capuchon de protection agencé sur son extrémité libre. Selon une autre particularité, le système comporte un module réunissant plusieurs capuchons de protection.

Selon une autre particularité, le système comporte, pour chaque puits traversant, un dispositif d'élution comprenant un réservoir destiné à recevoir un fluide d'élution à injecter dans chaque puits et un réceptacle destiné à récupérer ledit fluide d'élution, traversant la paroi poreuse du puits.

L'invention concerne également un procédé de préparation d'un échantillon biologique contenant des espèces biologiques selon la revendication 10, mis en oeuvre à l'aide d'un système automatisé tel que défini ci-dessus, le procédé comportant des étapes de :
- Commande des moyens d'entraînement pour positionner la plaque support dans une position de travail,
- Commande des moyens d'entraînement pour insérer en translation le premier organe mobile dans chaque puits à travers son premier accès jusqu'à venir en appui contre ladite paroi poreuse,
- Commande des moyens d'entraînement pour insérer en translation le deuxième organe mobile dans chaque puits à travers son deuxième accès jusqu'à venir en appui contre ladite paroi poreuse,
- Commande des moyens de stimulation de type mécanique et/ou thermique du premier organe mobile et/ou du deuxième organe mobile associés à chaque puits et/ou de la plaque support pour lyser les espèces biologiques.

### Brève description des figures

D'autres caractéristiques et avantages vont apparaître dans la description détaillée qui suit, faite en regard des dessins annexés dans lesquels :
- La figure 1 montre l'architecture simplifiée du système de l'invention ;
- La figure 2 représente une version améliorée du système de l'invention ;
- La figure 3 montre une architecture particulière du système de l'invention ;
- La figure 4 illustre les différentes étapes du procédé de préparation mises en oeuvre à l'aide du système conforme à l'invention ;

### Description détaillée d'au moins un mode de réalisation

Dans la suite de la description, les termes "haut", "bas", "supérieur", "inférieur" sont à comprendre en tenant compte d'un axe (A) tracé verticalement sur les figures annexées. L'invention vise un système automatisé permettant de réaliser une lyse mécanique d'un échantillon biologique avec une cadence élevée, et même de réaliser plusieurs lyses simultanément sur plusieurs échantillons en parallèle.

Le système se présente par exemple sous la forme d'un appareil de lyse d'échantillons biologiques pouvant fonctionner de manière totalement autonome.

Le système comporte une unité de contrôle UC chargée de contrôler différents modules (voir ci-après) du système pour mettre en oeuvre les différentes étapes du procédé de l'invention.

L'unité de contrôle peut comporter un automate programmable doté d'un module unité centrale et de plusieurs modules entrées/sorties.

Le système de l'invention comporte une plaque support M0.

Dans l'appareil, la plaque support M0 est avantageusement positionnée à l'horizontale. La plaque support M0 comporte un ou plusieurs puits 10 traversants, réalisés à travers son épaisseur. Sur les figures annexées, la plaque support M0 comporte plusieurs puits mais il faut comprendre que le principe de l'invention s'applique à un seul puits de la plaque.

Chaque puits 10 de la plaque M0 comporte un premier accès, dit accès supérieur, et un deuxième accès, dit accès inférieur.

Entre son accès supérieur et son accès inférieur, un puits 10 comporte une paroi poreuse 100 transversale, formant un filtre, entre sa partie supérieure et sa partie inférieure.

La paroi 100 peut être à surface lisse ou rugueuse du côté supérieur et/ou du côté inférieur. Les espèces biologiques Y à lyser sont placés contre la paroi rugueuse, pour faciliter et accélérer le broyage de ses espèces biologiques.

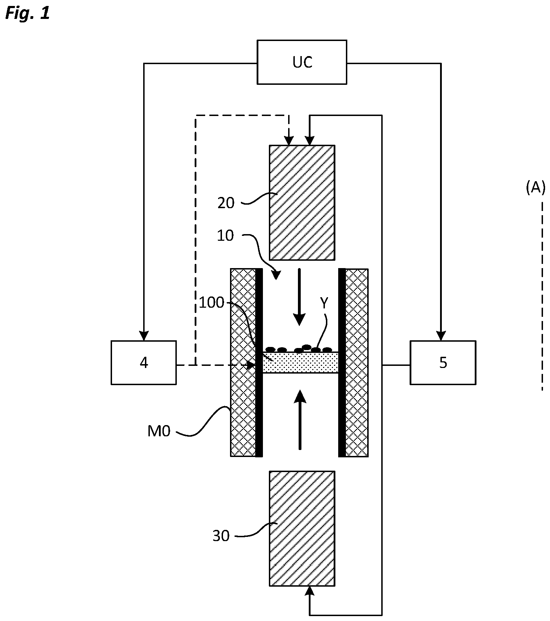

En référence à la figure 1, pour chaque puits 10 de la plaque support M0, le système comporte :
- Un premier organe mobile 20 actionnable en translation dans l'axe du puits 10 pour s'insérer à travers son accès supérieur,
- Un deuxième organe mobile 30, actionnable en translation dans l'axe du puits pour s'insérer à travers son accès inférieur,
- Des moyens de stimulation 4 mécanique et/ou thermique du premier organe mobile et/ou du deuxième organe mobile et/ou de la plaque support M0.

Le système comporte également des moyens d'entraînement 5 commandés par l'unité de contrôle UC. Ces moyens d'entraînement 5 peuvent comporter, des moteurs, des bras, des vérins ou équivalents, capables de déplacer les modules et autres du système selon divers axes et dans différents plans.

Les moyens d'entraînement 5 du système sont notamment configurés et agencés pour :
- Déplacer le premier organe mobile 20 et le deuxième organe mobile 30, dans des mouvements de translation (voir ci-après),
- Déplacer la plaque support M0 et la remplacer,
- Déplacer chaque module du système, selon l'étape du procédé qui est mise en oeuvre.

Il faut en effet noter que le système peut comporter plusieurs plaques supports M0, par exemple toutes identiques, présentes dans un espace de stockage et en attente d'être mises en position de travail. L'unité de contrôle UC est chargée de sélectionner une plaque support parmi l'ensemble des plaques supports disponibles dans l'espace de stockage et de commander des moyens d'entraînement 5 pour faire passer la plaque support sélectionnée de la position de stockage vers la position de travail.

Les moyens de dispense sont destinés à délivrer, dans le puits, l'échantillon biologique X à traiter. L'échantillon biologique X peut se présenter sous une forme liquide contenant des espèces biologiques Y. La partie liquide peut être évacuée en passant à travers la paroi poreuse du puits 10, ne laissant que les espèces biologiques Y à lyser sur la face supérieure de la paroi 100.

Ces moyens de dispense sont optionnels car la plaque support M0 pourrait être fournie préremplie avec un échantillon X à traiter présent dans chaque puits.

Pour l'ensemble des puits de la plaque support, les moyens de dispense peuvent être réunis en un seul module de dispense M1 comprenant plusieurs réservoirs 70.

Les moyens d'entraînement 5 sont configurés pour placer le module de dispense M1 au-dessus de la plaque support M0, en positionnant chaque réservoir en vis-à-vis d'un puits 10 distinct pour délivrer un échantillon X dans chaque puits.

Une fois que les échantillons X sont injectés dans chaque puits 10 de la plaque support M0, le module de dispense M1 est retiré, par commande des moyens d'entraînement 5.

Pour chaque puits 10, le système comporte un premier organe mobile 20 et un deuxième organe mobile 30, utilisés pour la lyse des espèces biologiques Y présentes dans le puits 10.

Le premier organe mobile 20 vient s'insérer en translation dans le puits par son accès supérieur et le deuxième organe mobile 30 vient s'insérer en translation dans le puits par son accès inférieur. La paroi 100 transversale du puits 10 peut ainsi être enserré entre les deux organes mobiles.

Le premier organe mobile 20 et le deuxième organe mobile 30 peuvent chacun se présenter sous la forme d'une tige rigide présentant une première extrémité fixée à un support mobile et une extrémité opposée qui est libre.

L'extrémité libre de l'organe mobile destiné à venir lyser les espèces biologiques peut être structurée ou non, pour faciliter l'opération de broyage.

L'ensemble des premiers organes mobiles peut être réuni en un seul module M2 actionnable pour tous les puits de la plaque support.

De même, l'ensemble des deuxièmes organes mobiles peut être réuni en un seul module M3 actionnable pour tous les puits de la plaque support.

Les moyens d'entraînement 5 sont configurés pour positionner chacun des deux modules M2, M3, de sorte que leurs organes mobiles se trouvent en vis-à-vis d'un puits distinct de la plaque support. Les moyens d'entraînement 5 sont ensuite configurés pour entraîner chaque organe mobile en translation de manière à ce que, pour chaque puits :
- Le premier organe mobile vienne en appui par son extrémité libre contre la face supérieure de la paroi transversale du puits,
- Le deuxième organe mobile vienne en appui par son extrémité libre contre la face inférieure de la paroi transversale du puits.

Les deux organes mobiles permettent de créer une solution mécaniquement stable, dans laquelle l'organe mobile inférieur crée un appui pour l'organe mobile supérieur lors de la lyse.

Pour chaque puits, le système comporte des moyens de stimulation du premier organe mobile et/ou du deuxième organe mobile et/ou de la plaque support M0. Ces moyens de stimulation sont destinés à permettre la lyse des espèces biologiques présentes dans l'échantillon biologique.

De manière non limitative, les moyens de stimulation 4 peuvent être de type mécanique et/ou thermique.

Les moyens de stimulation 4 mécanique peuvent être de type vibratoire. Dans ce cas, la vibration peut être appliquée à l'organe mobile destiné à être en contact avec les espèces biologiques Y à lyser. Il est également possible de maintenir les deux organes mobiles dans une position fixe et de mettre la plaque support en vibration.

On vient ainsi actionner au moins un organe mobile et/ou la plaque support M0. Il est aussi possible d'actionner tout en même temps. Il faut noter que la lyse peut se produire par mouvement relatif. La stimulation peut être appliquée à l'organe mobile directement en contact avec les espèces biologiques Y, ou par stimulation de l'autre organe mobile, celui-ci coopérant avec la plaque support M0 pour l'entraîner en mouvement, ou par stimulation de la plaque support par rapport aux deux organes mobiles fixes.

Il est également possible d'actionner l'organe mobile en contact avec les espèces biologiques pour lui conférer un mouvement de rotation autour d'une liaison rotule de sa première extrémité.

Les moyens de stimulation 4 de type thermique consistent par exemple à chauffer l'extrémité libre de l'organe mobile venant en contact avec les espèces biologiques. Le système peut comporter un élément chauffant commun au module de lyse, la chaleur se diffusant jusqu'à l'extrémité libre de chaque organe mobile. On peut aussi équiper chaque organe mobile d'un élément chauffant indépendant et contrôlable, par exemple une mini-résistance chauffante, un module à effet Peltier.

Dans ces différents cas, pour accélérer la lyse, la face de la paroi transversale 100 du puits 10 supportant les espèces biologiques Y est avantageusement rugueuse.

Le système peut comporter des moyens d'élution du matériel biologique obtenu dans chaque puits après la lyse. Pour chaque puits 10, ces moyens d'élution peuvent comporter un réservoir 80 de liquide d'élution destiné à être placé au-dessus du puits, et un réceptacle 81 destiné à recevoir le liquide après élution, situé sous le puits. De même que précédemment, le système peut comporter un module M4 réunissant plusieurs réservoirs de liquide d'élution dans une même structure et un module M5 réunissant les réceptacles. Les réservoirs présents dans le module M4 et les réceptacles présents dans le module M5 peuvent être communs à plusieurs puits 10 de la plaque support M0. Ces deux modules, dits modules d'élution, sont susceptibles d'être mis en position, respectivement au-dessus et au-dessous de la plaque support lors de l'élution du matériel biologique. Les moyens d'entraînement, commandés par l'unité de contrôle, sont configurés pour positionner chacun des deux modules d'élution M4, M5 dans leur position de travail. Le module M4 comporte également un système de vanne commandé par l'unité de contrôle et destiné à contrôler la libération du liquide d'élution stocké dans chaque réservoir du module M4, à destination de chaque puits de la plaque support.

Pour chaque organe mobile destiné à rentrer en contact avec les espèces biologiques, le système peut comporter un capuchon de protection 90 à adapter sur son extrémité libre. Il est possible de prévoir un module de protection M6, réunissant l'ensemble des capuchons 90 en un seul élément, et destiné à venir s'adapter sur le module M2 ou M3 de support des organes mobiles. Les moyens d'entraînement sont également configurés pour venir adapter ce module de protection M6.

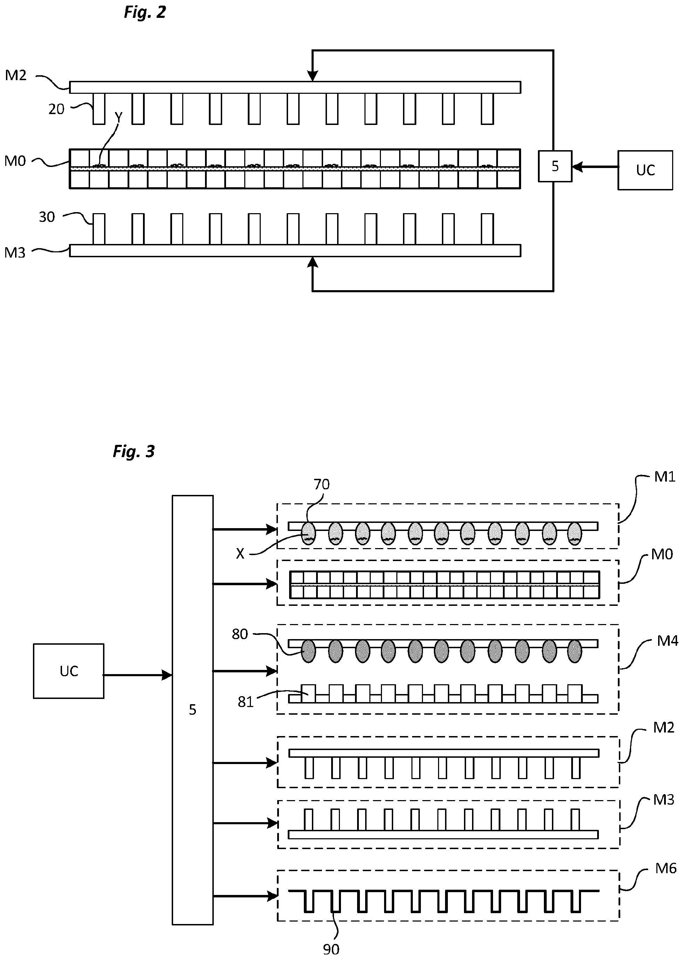

La figure 3 reprends de manière schématique les différents modules M0 à M6 de l'invention, montrant qu'ils sont actionnables par les moyens d'entraînement 5, via des commandes de l'unité de contrôle UC.

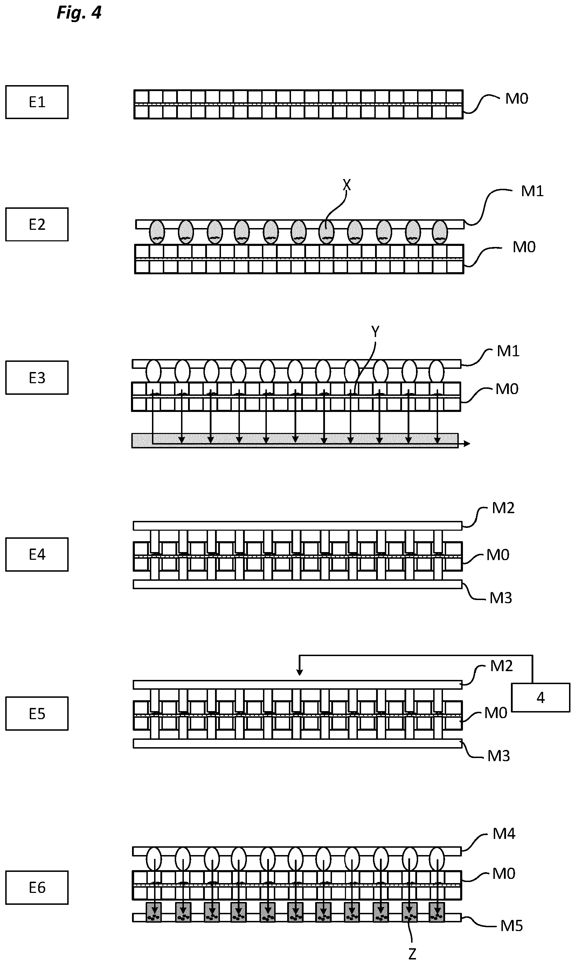

En référence à la figure 4, le système peut fonctionner de la manière suivante :
- E1 : L'unité de contrôle UC sélectionne une plaque support M0 et commande les moyens d'entraînement 5 pour positionner la plaque support sélectionnée dans la position de travail, à l'horizontale.
- E2 : L'unité de contrôle commande les moyens d'entraînement 5 pour positionner le module de dispense au-dessus de la plaque support.
- E3 : L'unité de contrôle commande le module de dispense M1 pour délivrer un échantillon biologique X dans chaque puits de la plaque support M0 et le faire traverser à travers la paroi poreuse de chaque puits. Le liquide filtré est évacué.

- L'unité de contrôle commande les moyens d'entraînement 5 pour retirer le module de dispense.
- L'unité de contrôle commande les moyens d'entraînement 5 pour placer les modules de lyse M2, M3.
- E4 : L'unité de contrôle commande les moyens d'entraînement 5 pour déplacer les modules de lyse M2, M3 en translation, de sorte qu'un premier organe mobile 20 vienne s'insérer dans chaque puits par son accès supérieur et qu'un deuxième organe mobile 30 vienne s'insérer dans chaque puits par son accès inférieur. Les deux organes mobiles viennent chacun en appui sur les deux faces opposées de la paroi transversale du puits.
- E5 : L'unité de contrôle commande les moyens de stimulation 4 pour lyser les espèces biologiques présentes dans chaque puits.
- Comme indiqué ci-dessus, la stimulation peut être de différents types, mécanique et/ou thermique, appliquée à un ou deux organes mobiles et/ou à la plaque support M0.
- Une fois la lyse terminée, l'unité de contrôle commande le retrait des modules de lyse M2, M3.
- L'unité de contrôle peut commander l'élution du matériel biologique obtenu dans chaque puits. L'unité de contrôle commande les moyens d'entraînement pour positionner les modules d'élution M4, M5, respectivement au-dessus et au-dessous de la plaque support.
- E6 : L'unité de contrôle commande l'injection d'un liquide d'élution dans chaque puits de la plaque support. Le liquide comportant le matériel biologique Z d'intérêt est récupéré dans chaque réceptacle du module M5.

Le système de l'invention présente de nombreux avantages, parmi lesquels :
- Il permet de réaliser la lyse d'un ou plusieurs échantillons en parallèle de manière totalement automatisée ;
- Il utilise un matériel classiquement employé dans le domaine de la manipulation d'échantillons biologiques ;

Il permet la lyse, sans aucune intervention humaine et en utilisant avantageusement du matériel à usage unique, limitant les risques de contamination.

## Revendications

1. Système automatisé de préparation d'un échantillon biologique (X) contenant des espèces biologiques (Y), **caractérisé en ce qu'**il comporte :
- Une plaque support (M0) dans laquelle sont réalisés un ou plusieurs puits (10) traversants,
- Chaque puits (10) traversant présentant deux accès opposés, un premier accès et un deuxième accès, le premier accès étant séparé du deuxième accès par une paroi poreuse (100), formant un filtre, contre laquelle peut être déposé ledit échantillon biologique à lyser,
- Et pour chaque puits :
∘ Un premier organe mobile (20), actionnable en translation dans l'axe du puits pour s'insérer à travers son premier accès,
∘ Un deuxième organe mobile (30), actionnable en translation dans l'axe du puits pour s'insérer à travers le deuxième accès,
- Des moyens d'entraînement (5) en translation de chaque premier organe mobile et de chaque deuxième organe mobile,
- Des moyens de stimulation (4) de type mécanique et/ou thermique de chaque premier organe mobile et/ou de chaque deuxième organe mobile et/ou de la plaque support,
- Une unité de contrôle (UC) configurée pour :
∘ Commander lesdits moyens d'entraînement,
∘ Commander lesdits moyens de stimulation.

2. Système selon la revendication 1, **caractérisé en ce que** les moyens d'entraînement (5) sont configurés pour déplacer ladite plaque support (M0) entre au moins deux positions distinctes, une position de stockage dans laquelle elle est en attente d'utilisation et une position de travail dans laquelle elle est positionnée pour recevoir dans chaque puits (10), un échantillon biologique (X).

3. Système selon la revendication 1 ou 2, **caractérisé en ce qu'**il comporte, pour chaque puits (10) de la plaque support (M0), des moyens de dispense d'un échantillon biologique (X) dans chaque puits de la plaque de support, les moyens d'entraînement (5) étant configurés pour déplacer les moyens de dispense entre deux positions distinctes, une position de stockage et une position de travail dans laquelle ils sont contrôlables pour injecter un échantillon biologique dans chaque puits.

4. Système selon l'une des revendications 1 à 3, **caractérisé en ce que** les moyens de stimulation (4) de type mécanique comprennent des moyens d'actionnement de type vibratoire coopérant avec le premier organe mobile (20) et/ou le deuxième organe mobile (30).

5. Système selon l'une des revendications 1 à 3, **caractérisé en ce que** les moyens de stimulation (4) de type mécanique comprennent des moyens d'actionnement en rotation autour de son axe du premier organe mobile et/ou du deuxième organe mobile.

6. Système selon l'une des revendications 1 à 5, **caractérisé en ce que** les moyens de stimulation (4) de type thermique comportent un élément chauffant intégré dans le premier organe mobile et/ou le deuxième organe mobile.

7. Système selon l'une des revendications 1 à 6, **caractérisé en ce que** le premier organe mobile (20) et/ou le deuxième organe mobile (30) présente une extrémité libre et **en ce que** le système comporte pour le premier organe mobile et/ou le deuxième organe mobile un capuchon de protection (90) agencé sur son extrémité libre.

8. Système selon la revendication 7, **caractérisé en ce qu'**il comporte un module (M6) réunissant plusieurs capuchons de protection (90).

9. Système selon l'une des revendications 1 à 8, **caractérisé en ce qu'**il comporte, pour chaque puits (10) traversant, un dispositif d'élution comprenant un réservoir (80) destiné à recevoir un fluide d'élution à injecter dans chaque puits et un réceptacle (81) destiné à récupérer ledit fluide d'élution, traversant la paroi poreuse (100) du puits.

10. Procédé de préparation d'un échantillon biologique (X) contenant des espèces biologiques (Y), mis en oeuvre à l'aide d'un système automatisé tel que défini dans l'une des revendications 1 à 9, **caractérisé en ce qu'**il comporte des étapes de :
- Commande des moyens d'entraînement (5) pour positionner la plaque support (M0) dans une position de travail,
- Commande des moyens d'entraînement (5) pour insérer en translation le premier organe mobile (20) dans chaque puits à travers son premier accès jusqu'à venir en appui contre ladite paroi poreuse,
- Commande des moyens d'entraînement pour insérer en translation le deuxième organe mobile (30) dans chaque puits à travers son deuxième accès jusqu'à venir en appui contre ladite paroi poreuse,
- Commande des moyens de stimulation (4) de type mécanique et/ou thermique du premier organe mobile et/ou du deuxième organe mobile associés à chaque puits et/ou de la plaque support (M0) pour lyser les espèces biologiques (Y).

## Patentansprüche

1. Automatisiertes System zur Vorbereitung einer biologischen Probe (X), die biologische Spezies (Y), enthält, **dadurch gekennzeichnet, dass** es umfasst:
- Eine Trägerplatte (M0), in der ein oder mehrere durchgehende Schächte (10) ausgeführt sind,
- Wobei jeder durchgehende Schacht (10) zwei entgegengesetzte Zugänge aufweist, einen ersten Zugang und einen zweiten Zugang, wobei der erste Zugang von dem zweiten Zugang durch eine einen Filter bildende poröse Wand (100) getrennt wird, auf welche die zu lysierende biologische Probe aufgebracht werden kann,
- Und für jeden Schacht:
∘ Ein erstes bewegliches Organ (20), das in der Achse des Schachts translatorisch betätigbar ist, um durch dessen ersten Zugang hindurch eingeführt zu werden,
∘ Ein zweites bewegliches Organ (30), das in der Achse des Schachts translatorisch betätigbar ist, um durch dessen zweiten Zugang hindurch eingeführt zu werden,
- Antriebsmittel (5) zum translatorischen Antreiben jedes ersten beweglichen Organs und jedes zweiten beweglichen Organs,
- Anregungsmittel (4) vom mechanischen und/oder thermischen Typ zur Anregung jedes ersten beweglichen Organs und/oder jedes zweiten beweglichen Organs und/oder der Trägerplatte,
- Eine Steuereinheit (UC), die dazu ausgestaltet ist:
∘ Die Antriebsmittel zu steuern,
∘ Die Anregungsmittel zu steuern.

2. System nach Anspruch 1, **dadurch gekennzeichnet, dass** die Antriebsmittel (5) dazu ausgestaltet sind, die Trägerplatte (M0) zwischen mindestens zwei verschiedenen Positionen zu bewegen, einer Lagerposition, in der sie auf die Verwendung wartet, und einer Arbeitsposition, in der sie dazu positioniert ist, in jedem Schacht (10) eine biologische Probe (X) aufzunehmen.

3. System nach Anspruch 1 oder 2, **dadurch gekennzeichnet, dass** es, für jeden Schacht (10) der Trägerplatte (M0), Abgabemittel zum Abgeben einer biologischen Probe (X) in jeden Schacht der Trägerplatte umfasst, wobei die Antriebsmittel (5) dazu ausgestaltet sind, die Abgabemittel zwischen zwei verschiedenen Positionen zu bewegen, einer Lagerposition und einer Arbeitsposition, in der sie ansteuerbar sind, um eine biologische Probe in jeden Schacht zu injizieren.

4. System nach einem der Ansprüche 1 bis 3, **dadurch gekennzeichnet, dass** die Anregungsmittel (4) vom mechanischen Typ Betätigungsmittel vom vibratorischen Typ aufweisen, die mit dem ersten beweglichen Organ (20) und/oder dem zweiten beweglichen Organ (30) zusammenwirken.

5. System nach einem der Ansprüche 1 bis 3, **dadurch gekennzeichnet, dass** die Anregungsmittel (4) vom mechanischen Typ Betätigungsmittel aufweisen, die um seine Achse des ersten beweglichen Organs und/oder des zweiten beweglichen Organs rotieren.

6. System nach einem der Ansprüche 1 bis 5, **dadurch gekennzeichnet, dass** die Anregungsmittel (4) vom thermischen Typ ein Heizelement umfassen, das in das erste bewegliche Organ und/oder das zweite bewegliche Organ integriert ist.

7. System nach einem der Ansprüche 1 bis 6, **dadurch gekennzeichnet, dass** das erste bewegliche Organ (20) und/oder das zweite bewegliche Organ (30) ein freies Ende aufweist und dass das System für das erste bewegliche Organ und/oder das zweite bewegliche Organ eine Schutzkappe (90) aufweist, die an seinem freien Ende angeordnet ist.

8. System nach Anspruch 7, **dadurch gekennzeichnet, dass** es ein Modul (M6) umfasst, das mehrere Schutzkappen (90) vereint.

9. System nach einem der Ansprüche 1 bis 8, **dadurch gekennzeichnet, dass** es, für jeden durchgehenden Schacht (10), eine Elutionsvorrichtung umfasst, aufweisend einen Behälter (80), der dazu bestimmt ist, ein in jeden Schacht zu injizierendes Elutionsfluid aufzunehmen, und ein Behältnis (81), das dazu bestimmt ist, das Elutionsfluid zurückzugewinnen, das die poröse Wand (100) des Schachts durchquert hat.

10. Verfahren zur Vorbereitung einer biologischen Probe (X), die biologische Spezies (Y) enthält, das mithilfe eines automatisierten Systems wie in einem der Ansprüche 1 bis 9 definiert durchgeführt wird, **dadurch gekennzeichnet, dass** es Schritte umfasst zum:
- Steuern der Antriebsmittel (5), um die Trägerplatte (M0) in einer Arbeitsposition zu positionieren,
- Steuern der Antriebsmittel (5), um das erste bewegliche Organ (20) translatorisch in jeden Schacht durch dessen ersten Zugang hindurch einzuführen, bis es an der porösen Wand zur Anlage kommt,
- Steuern der Antriebsmittel, um das zweite bewegliche Organ (30) translatorisch in jeden Schacht durch dessen zweiten Zugang hindurch einzuführen, bis es an der porösen Wand zur Anlage kommt,
- Steuern der Anregungsmittel (4) vom mechanischen und/oder thermischen Typ des ersten beweglichen Organs und/oder des zweiten beweglichen Organs, die jedem Schacht zugeordnet sind, und/oder der Trägerplatte (M0), um die biologischen Spezies (Y) zu lysieren.

## Claims

1. Automated system for preparing a biological sample (X) containing biological species (Y), **characterized in that** it comprises:
- a support plate (M0) in which one or more through-wells (10) are made,
- each through-well (10) having two opposite accesses, a first access and a second access, the first access being separated from the second access by a filter-forming porous wall (100), against which the said biological sample that is to undergo lysis may be placed,
- and for each well:
∘ a first mobile member (20), that can be actuated to move translationally along the axis of the well so as to become inserted across the first access thereof,
∘ a second mobile member (30), that can be actuated to move translationally along the axis of the well so as to become inserted across the second access thereof,
- drive means (5) driving the translational movement of each first mobile member and of each second mobile member,
- stimulation means (4) of mechanical and/or thermal type for stimulating each first mobile member and/or each second mobile member and/or the support plate,
- a control unit (UC), configured to:
∘ command said drive means,
∘ command said stimulation means.

2. System according to Claim 1, **characterized in that** the drive means (5) are configured to move said support plate (M0) between at least two distinct positions: a storage position in which it is on standby awaiting use and a working position in which it is positioned to receive a biological sample (X) in each well (10).

3. System according to Claim 1 or 2, **characterized in that** it comprises, for each well (10) of the support plate (M0), means for dispensing a biological sample (X) into each well of the support plate, the drive means (5) being configured to move the dispensing means between two distinct positions: a storage position and a working position in which they can be controlled such that they inject a biological sample into each well.

4. System according to one of Claims 1 to 3, **characterized in that** the mechanical-type stimulation means (4) comprise actuating means of vibratory type collaborating with the first mobile member (20) and/or the second mobile member (30).

5. System according to one of Claims 1 to 3, **characterized in that** the mechanical-type stimulation means (4) comprise actuating means causing the first mobile member and/or the second mobile member to rotate about its axis.

6. System according to one of Claims 1 to 5, **characterized in that** the thermal-type stimulation means (4) comprise a heating element incorporated into the first mobile member and/or the second mobile member.

7. System according to one of Claims 1 to 6, **characterized in that** the first mobile member (20) and/or the second mobile member (30) has a free end and **in that** the system comprises, for the first mobile member and/or the second mobile member, a protective cap (90) arranged over the free end thereof.

8. System according to Claim 7, **characterized in that** it comprises a module (M6) combining a plurality of protective caps (90).

9. System according to one of Claims 1 to 8, **characterized in that** it comprises, for each through-well (10), an elution device comprising a reservoir (80) intended to receive an elution fluid that is to be injected into each well, and a receptacle (81) intended to recover said elution fluid, passing through the porous wall (100) of the well.

10. Method for preparing a biological sample (X) containing biological species (Y), implemented using an automated system such as defined in one of Claims 1 to 9, **characterized in that** it comprises the steps of:
- commanding the drive means (5) to position the support plate (M0) in a working position,
- commanding the drive means (5) to insert the first mobile member (20) translationally into each well across the first access thereof until it comes to bear against said porous wall,
- commanding the drive means to insert the second mobile member (30) translationally into each well across the second access thereof until it comes to bear against said porous wall,
- commanding the mechanical-type and/or thermal-type stimulation means (4) of the first mobile member and/or second mobile member associated with each well and/or of the support plate (M0) to bring about lysis of the biological species (Y).
